# EUROPEAN PATENT APPLICATION

(11) **EP 1 344 497 A1**
(43) Date of publication of application: **17.09.2003**
(21) Application number: 03076237.1
(22) Date of filing: 15.08.1996
(51) Int. Cl.: A61B 18/14, A61N 1/40, A61N 5/04

(54) **Rf apparatus for the ablation of selected mass**

(30) Priority: 15.08.1995 US 515379
(62) Divisional of application: 96931384.0
(71) Applicant: Rita Medical Systems, Inc., Mountain View, CA 94043 (US)
(72) Inventor: Gough, Edward J., Menlo Park, CA 94025 (US); Stein, Alan A., Moss Beach, CA 94038 (US)
(74) Representative: Potter, Julian Mark

(57) **Abstract**

An ablation treatment apparatus has a multiple probe device. The multiple probe device includes a primary electrode/probe/antenna with at least one lumen, a longitudinal axis and an ablative surface area of length L₁. The multiple probe device also includes a secondary electrode/probe/antenna that is positioned in a lumen of the primary probe as this last is introduced through tissue. A secondary electrode/probe/antenna distal end is slidingly deployed at a selected tissue site from the primary electrode/probe/antenna lumen in a lateral direction relative to the longitudinal axis. A sensor is at least partially positioned at an exterior of the secondary electrode/probe/antenna along the secondary electrode/probe/antenna distal end. L₂ is at least equal to 1/3 L₁. An energy source is coupled to the primary electrode/probe/antenna.

## Description

### Reference to Related Application

This application claims priority to U.S. Patent Application No. 08/515,379, filed August 15, 1995, entitled "Multiple Antenna Ablation Apparatus", by Gough, et al., incorporated herein by reference.

### BACKGROUND OF THE INVENTION

### Field of the Invention

This invention relates generally to an apparatus for the treatment and ablation of body masses, such as tumors, and more particularly, to an apparatus with a plurality of electrodes.

### Description of the Related Art

Current open procedures for treatment of tumors are extremely disruptive and cause a great deal of damage to healthy tissue. During the surgical procedure, the physician must exercise care in not cutting the tumor in a manor that creates seeding of the tumor, resulting in metastasis. In recent years, development of products has been directed with an emphasis on minimizing the traumatic nature of traditional surgical procedures.

There has been a relatively significant amount of activity in the area of hyperthermia as a tool for treatment of tumors. It is known that elevating the temperature of tumors is helpful in the treatment and management of cancerous tissues. The mechanisms of selective cancer cell eradication by hyperthermia are not completely understood. However, four cellular effects of hyperthermia on cancerous tissue have been proposed, (i) changes in cell or nuclear membrane permeability or fluidity, (ii) cytoplasmic lysomal disintegration, causing release of digestive enzymes, (iii) protein thermal damage affecting cell respiration and the synthesis of DNA or RNA and (iv) potential excitation of immunologic systems. Treatment methods for applying heat to tumors include the use of direct contact radio-frequency (RF) applicators, microwave radiation, inductively coupled RF fields, ultrasound, and a variety of simple thermal conduction techniques.

Among the problems associated with all of these procedures is the requirement that highly localized heat be produced at depths of several centimeters beneath the surface of the skin. Certain techniques have been developed with microwave radiation and ultrasound to focus energy at various desired depths. RF applications may be used at depth during surgery. However, the extent of localization is generally poor, with the result that healthy tissue may be harmed. Induction heating gives rise to poor localization of the incident energy as well. Although induction heating may be achieved by placing an antenna on the surface of the body, superficial eddy currents are generated in the immediate vicinity of the antenna, when it is driven using RF current, and unwanted surface heating occurs with little heat delivered to the underlying tissue.

Thus, non-invasive procedures for providing heat to internal tumors have had difficulties in achieving substantial specific and selective treatment.

Hyperthermia, which can be produced from an RF or microwave source, applies heat to tissue, but does not exceed 45 degrees C, so that normal cells survive. In thermotherapy, heat energy of greater than 45 degrees C is applied resulting in histological damage, desiccation and the denaturization of proteins. Hyperthermia has been applied more recently for therapy of malignant tumors. In hyperthermia, it is desirable to induce a state of hyperthermia that is localized by interstitial current heating to a specific area while concurrently insuring minimum thermal damage to healthy surrounding tissue. Often, the tumor is located subcutaneously and addressing the tumor requires either surgery, endoscopic procedures or external radiation. It is difficult to externally induce hyperthermia in deep body tissue because current density is diluted due to its absorption by healthy tissue. Additionally, a portion of the RF energy is reflected at the muscle/fat and bone interfaces which adds to the problem of depositing a known quantity of energy directly on a small tumor.

Attempts to use interstitial local hyperthermia have not proven to be very successful. Results have often produced nonuniform temperatures throughout the tumor. It is believed that tumor mass reduction by hyperthermia is related to thermal dose. Thermal dose is the minimum effective temperature applied throughout the tumor mass for a defined period of time. Because blood flow is the major mechanism of heat loss for tumors being heated, and blood flow varies throughout the tumor, more even heating of tumor tissue is needed to ensure effective treatment.

The same is true for ablation of the tumor itself through the use of RF energy. Different methods have been utilized for the RF ablation of masses such as tumors. Instead of heating the tumor it is ablated through the application of energy. This process has been difficult to achieve due to a variety of factors including, (i) positioning of the RF ablation electrodes to effectively ablate all of the mass, (ii) introduction of the RF ablation electrodes to the tumor site and (iii) controlled delivery and monitoring of RF energy to achieve successful ablation without damage to non-tumor tissue.

There have been a number of different treatment methods and devices for minimally invasively treating tumors. One such example is an endoscope that produces PF hyperthermia in tumors, as disclosed in U.S. Patent No. 4,920,978. A microwave endoscope device is described in U.S. Patent No. 4,409,993. In U.S. Patent No. 4,920,978, an endoscope for RF hyperthermia is disclosed.

In U.S. Patent No. 4,763,671 (the "'671 patent"), a minimally invasive procedure utilizes two catheters that are inserted interstitially into the tumor. The catheter includes a hard plastic support member. Around the support member is a conductor in the form of an open mesh. A layer of insulation is secured to the conductor with adhesive beads. It covers all of the conductor except a preselected length which is not adjustable. Different size tumors cannot be treated with the same electrode. A tubular sleeve is introduced into the support member and houses radioactive seeds. The device of the '671 patent fails to provide for the introduction of a fluidics medium, such as a chemotherapeutic agent, to the tumor for improved treatment. The size of the electrode conductive surface is not variable. Additionally, the device of the '671 patent is not capable of maintaining a preselected level of power that is independent of changes in voltage or current.

In U.S. Patent No. 4,565,200 (the "'200 patent"), an electrode system is described in which a single entrance tract cannula is used to introduce an electrode into a selected body site. The device of the '200 patent is limited in that the single entrance tract fails to provide for the introduction, and removal of a variety of inserts, including but not limited to an introducer, fluid infusion device and insulation sleeve. Additionally, the device of the '200 patent fails to provide for the maintenance of a selected power independent of changes in current or voltage.

There is a need for a method and apparatus which is introduced into or adjacent to a tumor or other solid mass, and a plurality of electrodes are around the tumor or solid mass.

### SUMMARY OF THE INVENTION

Accordingly, it is an object of the invention to provide an ablation device that is introduced into a body and includes multiple electrodes or antennas.

Another object of the invention to provide an ablation device that is introduced into a body which includes multiple electrodes or antennas deployed from an introducer to surround a selected mass to be ablated.

Yet another object of the invention is to provide an ablation device with multiple deployed electrodes or antennas that includes feedback control.

Still a further object of the invention is to provide an ablation device with multiple deployed electrodes or antennas that includes one or more thermal sensors.

Another object of the invention is to provide an ablation device with multiple deployed electrodes or antennas that includes a cooling means.

Still another object of the invention is to provide an ablation device with multiple deployed electrodes or antennas which do not impede out.

These and other objectives are achieved in an ablation treatment apparatus with a multiple antenna device. The multiple antenna device includes a primary antenna with a lumen, a longitudinal axis and an ablative surface area of length L₁. The multiple antenna device also includes a secondary antenna that is positioned in the primary antenna as the primary antenna is introduced through tissue. A secondary antenna distal end is deployed at a selected tissue site from the primary antenna lumen in a lateral direction relative to the longitudinal axis. A sensor is at least partially positioned at an exterior of the secondary antenna distal end at a distance L₂, from the primary antenna along the secondary antenna distal end. L₂ is at least equal to 1/3 L₁. An energy source is coupled to the primary antenna.

### BRIEF DESCRIPTION OF THE FIGURES

Figure 1 is a cross-sectional view of the ablation apparatus of the present invention illustrating an electrode with a lumen, a cooling medium inlet conduit, a cooling medium outlet conduit and two probes extending from sidewall ports formed in the lumen.
Figure 2a is a cross-sectional view of the closed loop distal end of the two cooling medium conduits of Figure 1.
Figure 2b is a perspective view of a conic geometric ablation achieved with the apparatus of Figure 1.
Figure 2(c) is a cross-sectional view of the primary antenna with a closed distal end and a cooling element positioned in a central lumen of the primary antenna.
Figure 2(d) is a cross-sectional view of the primary antenna with an open distal end and a elongated cooling element positioned in the central lumen of the primary antenna.
Figure 2(e) is distal end view of the apparatus of Figure 2(d).
Figure 2(f) is a cross-sectional view of the apparatus of Figure 2(d) taken along the lines 2(f) - 2(f).
Figure 3a is a perspective view of the multiple antenna ablation apparatus of the present invention with two secondary antennas deployed into the selected tissue mass.
Figure 3b is a cross-sectional view of another embodiment of the closed loop distal end of the two cooling medium conduits.
Figure 4 is a cross-sectional of Figure 1 taken along the lines 4-4.
Figure 5 illustrates the creation of a 4 cm spherical ablation volume, with one sensor positioned at the periphery of the ablation volume, and a second sensor positioned on the probe midpoint between the electrode and the distal end of the probe.
Figure 6(a) is a perspective view of the ablation apparatus of the present invention illustrating two probes extending from a distal end of the electrode.
Figure 6(b) is a perspective view of the multiple antenna ablation of the present invention illustrating two antennas which provide a retaining and gripping function.
Figure 6(c) is a perspective view of the multiple antenna ablation of the present invention illustrating three secondary antennas which provide a retaining and gripping function.
Figure 6(d) is a cross-sectional view of the apparatus of Figure 6(c) taken along the lines 6(d)-6(d).
Figure 7 is a perspective view of the distal end of the electrode of the present invention with probes extending from a distal end of an insulation sleeve.
Figure 8 is a perspective view of the ablation apparatus of the present invention illustrating the deployment of four probes from the electrode.
Figure 9 is a block diagram illustrating the inclusion of a controller, energy source and other electronic components of the present invention.
Figure 10 is a cross-sectional view of the ablation apparatus of the present invention illustrating an electrode with a lumen, a cooling medium inlet conduit, a cooling medium outlet conduit and two probes extending from sidewall ports formed in the lumen.
Figure 11 is a cross-sectional view of the closed loop distal end of the two cooling medium conduits of Figure 10.
Figure 12 is a cross-sectional view of another embodiment of the closed loop distal end of the two cooling medium conduits.
Figure 13 is a cross-sectional of Figure 12 taken along the lines 4-4.
Figure 14 illustrates the creation of a 4 cm spherical ablation volume, with one sensor positioned at the periphery of the ablation volume, and a second sensor positioned on the probe midpoint between the electrode and the distal end of the probe.
Figure 15 is a block diagram illustrating a feedback system useful ton control the temperature of energy delivering electrodes.
Figure 16 illustrates a circuit useful to implement the feedback system of Figure 15.

### DETAILED DESCRIPTION

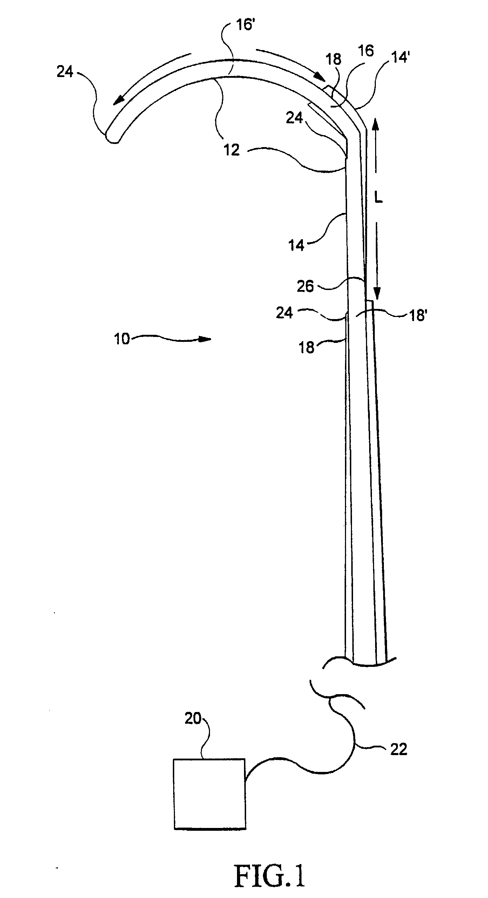

As shown in Figure 1, an ablation treatment apparatus 10 includes a multiple antenna device 12 of adjustable length. Multiple antenna device 12 includes a primary antenna 14 with an adjustable or non-adjustable energy delivery surface or length, and one or more secondary antennas 16 that are typically introduced from a lumen formed at least partially in primary antenna 14. Each secondary antenna 16 also has an adjustable or non-adjustable energy delivery surface or length. The adjustability of the lengths permits ablation of a wide variety of geometric configurations of a targeted mass. Lengths of primary and secondary antennas 14 and 16 can be adjusted, and primary antenna 14 is moved up and down, rotationally about its longitudinal axis, and back and forth, in order to define, along with sensors, the periphery or boundary of the ablated mass and ablate a variety of different geometries that are not always symmetrical.

Primary antenna or electrode 14 is constructed so that it can be introduced percutaneously or laparoscopicly into a solid mass. Primary antenna 14 can have a sharpened distal end 14' to assist introduction into the solid mass. Each secondary antenna 16 is constructed to be less structurally rigid than primary antenna 14. This is achieved by, (i) choosing different materials for antennas 14 and 16, (ii) using the same material but having less of it for secondary antenna 16, e.g., secondary antenna is not as thick as primary electrode 14, or including another material in one of the antennas 14 or 16 to vary their structural rigidity. For purposes of this disclosure, structural rigidity is defined as the amount of deflection that an antenna has relative to its longitudinal axis. It will be appreciated that a given antenna will have different levels of rigidity depending on its length. Primary and secondary antennas can be made of a variety of conductive materials, both metallic and non-metallic. One suitable material is type 304 stainless steel of hypodermic quality. The rigidity of primary antenna 14 is greater than secondary antenna 16. Depending on the application, the rigidity of secondary antenna 16 can be about 10%, 25%, 50%, 75% and 90% of the rigidity of primary antenna 14. Primary and secondary antennas 14 and 16 can be made of a variety of conductive materials, both metallic and nonmetallic. In some applications, secondary electrode 16 can be made of a shaped memory metal, such as NiTi, commercially available from Raychem Corporation, Menlo Park, California.

Each of primary or secondary antenna 14 or 16 can have different lengths. Suitable lengths include but are not limited to 17.5 cm, 25.0 cm. and 30.0 cm. The actual length of an antenna depends on the location of the targeted solid mass to be ablated, its distance from the skin, its accessibility as well as whether or not the physician chooses a laproscopic, percutaneous or other procedure. Further, ablation treatment apparatus 10, and more particularly multiple antenna device 12, can be introduced through a guide to the desired tissue mass site.

An insulation sleeve 18 is positioned around an exterior of one or both of the primary and secondary antennas 14 and 16 respectively. Preferably, each insulation sleeve 18 is adjustably positioned so that the length of antenna providing an ablation delivery surface can be varied. Each insulation sleeve 18 surrounding a primary antenna 14 can include one or more apertures. This permits the introduction of a secondary antenna 16 through primary antenna 14 and insulation sleeve 18. This distance that a secondary antenna 16 extends from insulation sleeve defines the length of distal end 16'.

One or more sensors 24 may be positioned on interior or exterior surfaces of primary antenna 14, secondary antenna 16 or insulation sleeve 18. Preferably sensors 24 are positioned at primary antenna distal end 14', secondary antenna distal end 16' and insulation sleeve distal end 18'. Secondary antenna 16 can extend from insulation sleeve distal end 18' in a lateral direction relative to primary antenna 14, and sensor 24 can be positioned at insulation sleeve distal end 18'. Preferably, sensor 24 is positioned at least partially on an exterior surface of distal end 16'.

L₁ is the length of the electromagnetic energy delivery surface of primary antenna 14. L₂ is the distance from primary antenna 14 to sensor 24 when sensor 24 is positioned at least partially on an exterior of distal end 16'. L₂ is measured from primary antenna 14 along the surface of distal end 16'. In various embodiments, the length of L₂ is at least equal to or greater than 33.33% of L₁, 50% of L₁, 75% or greater of L₁, at least equal to L₁ or is greater than L₁.

In one embodiment insulation sleeve can comprise a polyamide material, with a sensor positioned on top of the polyamide insulation, and a .002 inch shrink wrap. The polyamide insulating layer is semi-rigid. The sensor can lay down substantially the entire length of the polyamide.

The present invention provides a multiple antenna ablation apparatus. The apparatus includes an electromagnetic energy source, a trocar including a distal end, and a hollow lumen extending along a longitudinal axis of the trocar, and a multiple antenna ablation device with three or more antennas. The antennas are initially positioned in the trocar lumen as the trocar is introduced through tissue. At a selected tissue site the antennas are deployable from the trocar lumen in a lateral direction relative to the longitudinal axis. Each of the deployed antennas has an electromagnetic energy delivery surface of sufficient size to, (i) create a volumetric ablation between the deployed antennas, and (ii) create the volumetric ablation without impeding out any of the deployed antennas when 10 to 50 watts of electromagnetic energy is delivered from the electromagnetic energy source to the multiple antenna ablation device. At least one cable couples the multiple antenna ablation device to the electromagnetic energy source. For purposes of this specification the term "impeding out" means that a tissue area has become sufficiently desiccated or carbonized that the desiccated or carbonized tissue area has a resultant high electrical resistance that impairs the process of creating a coagulating lesion.

An energy source 20 is connected with multiple antenna device 12 with one or more cables 22. Energy source 20 can be an RF source, microwave source, short wave source, coherent and incoherent or ultrasound source, and the like. Multiple antenna device 12 can be comprised of primary and secondary antennas 14 and 16 that are RF antennas, microwave antennas, as well as combinations thereof. In one embodiment, energy source 20 is a combination RF/microwave box. Further a laser optical fiber, coupled to a laser source 20 can be introduced through one or both of primary or secondary antennas 14 and 16. One or more of the primary or secondary antennas 14 and 16 can be an arm for the purposes of introducing the optical fiber.

Antennas 14 and 16 are each electromagnetically coupled to energy source 20. The coupling can be direct from energy source 20 to each antenna 14 and 16, or indirect by using a collet, sleeve and the like which couples antennas 14 and 16 to energy source 20.

One or more sensors 24 are positioned on interior or exterior surfaces of primary antenna 14, secondary antenna 16 or insulation sleeve 18. Preferably sensors 24 are positioned at primary antenna distal end 14', secondary antenna distal end 16' and insulation sleeve distal end 18'. Sensors 24 permit accurate measurement of temperature at a tissue site in order to determine, (i) the extent of ablation, (ii) the amount of ablation, (iii) whether or not further ablation is needed and (iv) the boundary or periphery of the ablated mass. Further, sensors 24 prevent non-targeted tissue from being destroyed or ablated.

Sensors 24 are of conventional design, including but not limited to thermistors, thermocouples, resistive wires, and the like. Suitable thermal sensors 24 include a T type thermocouple with copper constantene, J type, E type, K type, fiber optics, resistive wires, thermocouple IR detectors, and the like. It will be appreciated that sensors 24 need not be thermal sensors.

Sensors 24 measure temperature and/or impedance to permit monitoring and a desired level of ablation to be achieved without destroying too much tissue. This reduces damage to tissue surrounding the targeted mass to be ablated. By monitoring the temperature at various points within the interior of the selected mass, a determination of the tumor periphery can be made, as well as a determination of when ablation is complete. If at any time sensor 24 determines that a desired ablation temperature is exceeded, then an appropriate feedback signal is received at energy source 20 which then regulates the amount of energy delivered to primary and/or secondary antennas 14 and 16.

Thus the geometry of the ablated mass is selectable and controllable. Any number of different ablation geometries can be achieved. This is a result of having variable lengths for primary antenna 14 and secondary antenna 16 ablation surfaces as well as the inclusion of sensors 24.

Preferably, secondary antenna 16 is laterally deployed out of an aperture 26 formed in primary antenna 14. Aperture 26 is typically positioned along the longitudinal axis of primary antenna 14.

Initially, primary antenna 14 is introduced into or adjacent to a target solid mass. Secondary antenna 16 is then introduced out of aperture 26 into the solid mass. There is wide variation in the amount of deflection of secondary antenna 16. For example, secondary antenna 16 can be deflected a few degrees from the longitudinal axis of primary antenna 14, or secondary antenna can be deflected in any number of geometric configurations, including but not limited to a "7" hook. Further, secondary antenna 16 is capable of being introduced from primary antenna 14 a few millimeters from primary antenna, or a much larger distance. Ablation by secondary antenna 16 can begin a few millimeters away from primary antenna 14, or secondary electrode 16 can be advanced a greater distance from primary antenna 14 and at that point the initial ablation by secondary antenna 16 begins.

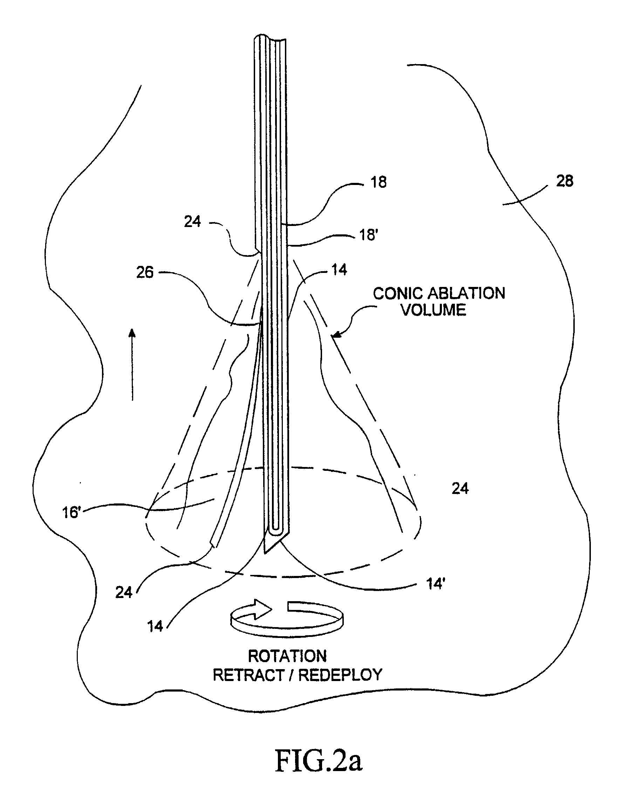

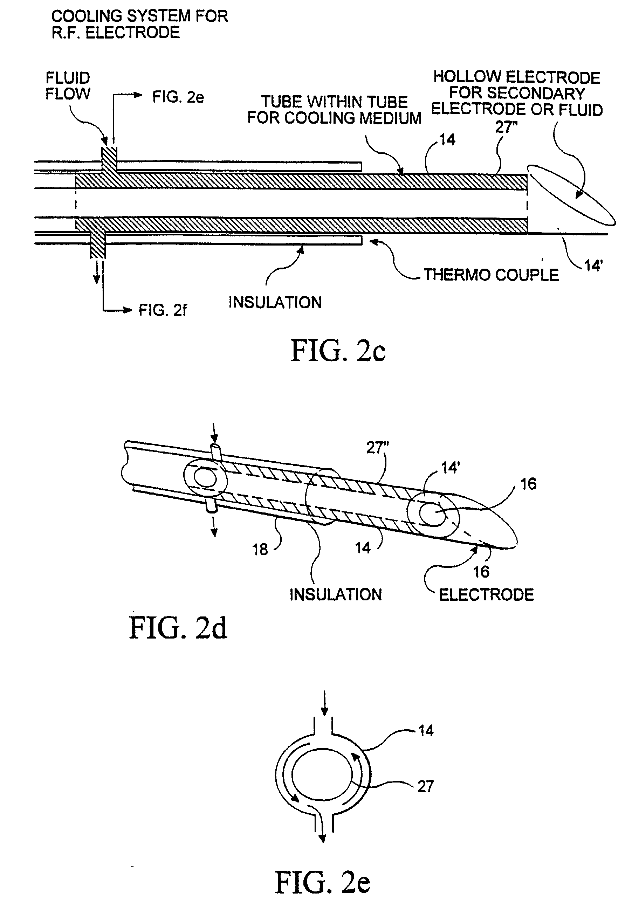

As illustrated in Figure 2(a), primary antenna 14 has been introduced into a selected tissue mass or tumor 28. Subsequently, secondary antenna distal end 16' is advanced out of aperture 26 and into selected tissue mass 28. Sensor 24 is positioned on distal end 16'. Insulation sleeves 18 can be included and may be fixed or adjusted. RF, microwave, short wave and the like energy is delivered to primary antenna 14, secondary antenna 16, or only to one. Either antenna 14 or 16 can be active or passive. In secondary antenna is active, then primary antenna distal end 14' includes a sensor 24. In this embodiment, L₁ is the length of the electromagnetic energy delivery surface of distal end 16', and L₂ is the distance from the tip of distal end 14' to the port positioned in primary antenna 14 from which secondary antenna 16 laterally extends. Antennas 14 and 16 can be operated in a monopolar mode (RF), or alternatively. multiple antenna device 12 can be operated in a bipolar mode (RF). Multi antenna device 12 can be switched between monopolar and bipolar operation and has multiplexing capability between antennas 14 and 16. Secondary antenna distal end 16' is retracted back into primary antenna 14, and primary antenna is then rotated. Secondary antenna distal end 16' is then introduced into selected tissue mass 28. Secondary antenna may be introduced a short distance into selected tissue mass 28 to ablate a small area. It can then be advanced further into any number of times to create more ablation zones. Again, secondary antenna distal end 16' is retracted back into primary antenna 14.

As also illustrated in Figure 2(a), primary antenna 14 has been introduced into a selected tissue mass 28. Subsequently, secondary antenna distal end 16' is advanced out of aperture 26 and into selected tissue mass 28. Sensor 24 is positioned on distal end 16'. Insulation sleeves 18 can be included and may be fixed or adjusted. RF, microwave, short wave and the like energy is delivered to primary antenna 14, secondary antenna 16, or only to one. Either antenna 14 or 16 can be active or passive. In secondary antenna is active, then primary antenna distal end 14' includes a sensor 24. In this embodiment, L₁ is the length of the electromagnetic energy delivery surface of distal end 16', and L₂ is the distance from the tip of distal end 14' to the port positioned in primary antenna 14 from which secondary antenna 16 laterally extends. Antennas 14 and 16 can be operated in a monopolar mode (RF), or alternatively, multiple antenna device 12 can be operated in a bipolar mode (RF). Multi antenna device 12 can be switched between monopolar and bipolar operation and has multiplexing capability between antennas 14 and 16. Secondary antenna distal end 16' is retracted back into primary antenna 14, and primary antenna is then rotated. Secondary antenna distal end 16' is then introduced into selected tissue mass 28. Secondary antenna may be introduced a short distance into selected tissue mass 28 to ablate a small area. It can then be advanced further into any number of times to create more ablation zones. Again, secondary antenna distal end 16' is retracted back into primary antenna 14

It can then be advanced further into tumor 28 any number of times to create more ablation zones. Again, secondary antenna 16 is retracted back into primary antenna 14, and primary antenna 14 can be, (i) rotated again, (ii) moved along a longitudinal axis of tumor 28 to begin another series of ablations with secondary antenna 16 being introduced and retracted in and out of primary antenna 14, or (iii) removed from tumor 28. A number of parameters permit ablation of tumors, masses of different sign and shapes including a series of ablations having primary and secondary antennas 14 and 16 with variable length ablation surfaces and the use of sensor 24.

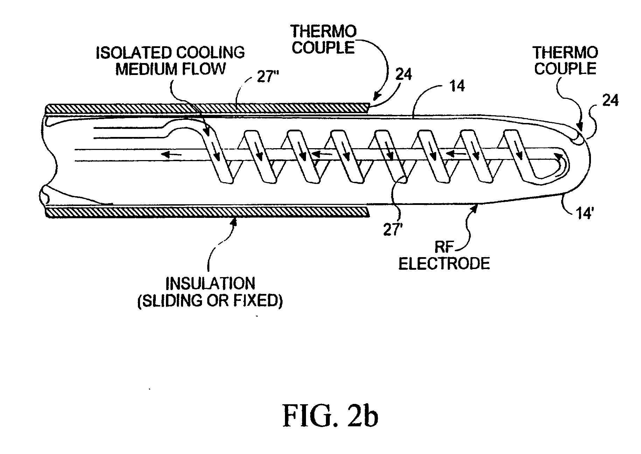

As illustrated in Figure 2(b), primary antenna 14 can include one or more cooling elements 27. One embodiment of a suitable cooling element 27 is a closed elongated structure 27' coupled to a circulating system to introduce a cooling element Two lumens can be incorporated with primary antenna 14, or secondary antenna 16, to carry a cooling medium to and away from antennas 14 or 16. In one embodiment, the dimensions are the lumens are; outer lumen 0.117 inches outer diameter by 0.088 inches inner diameter, and inner lumen 0.068 inches outer diameter by 0.060 inner diameter. The cooling medium enters primary antenna 14, absorbs heat generated in the tissue surrounding primary antenna 14, and the heated medium then exits antenna 14. This may be achieved by the use of the two lumens, one introducing the cooling medium, and the other lumen removing heated cooling solution. Heat is subsequently removed from the heated medium, and once again cooled medium is recirculated through antenna 14. This is a continuous process. Cooling element 27 need only be positioned, and provide the cooling function, along that section of antenna 14 which has an ablation energy delivery surface. Insulation sleeve 18 can be slideably adjustable along the length of primary antenna 14 or be in a fixed positioned. The exterior of primary antenna 14 which is not covered by insulation sleeve 18 provides the ablation energy delivery surface. It is only this surface which becomes heated and charred from electromagnetic energy delivered to adjacent tissue. Thus it is only necessary to cool this surface of antenna 14, and the actual cooling by cooling medium 17 can be limited to the ablation energy delivery surface.

Cooling medium may be a refrigerant including but not limited to ethyl alcohol, freon, polydimethlsiloxane, and the like. Cooling can also be achieved with gas expansion cooling by the Joule-Thompson effect.

In another embodiment of cooling element 27, distal end 14' is again closed, and a cooling medium 27 flows through the central lumen formed in antenna 14. The cooling medium 27 is coupled to a recirculation system, which may be a heat exchanger with a pump. The rate of fluid flow through primary antenna 14 is variable based on a number of different parameters.

In yet another embodiment, cooling element 27 is an elongated structure 27", including but not limited to a tubular member such as a cylinder, with a cooling medium flowing through elongated structure 27" (Figure 2(c)). Elongated structure 27" is positioned within the central lumen of primary antenna 14 and can extend to distal end 14'. Distal end 14' can be open or closed. Cooling medium is confined within elongated structure 27". This permits the introduction and flow of other mediums through the hollow lumen of primary antenna 14. Secondary antennas 16 can exit at distal end 14', or alternatively, they may also exit along a side of antenna 14 (Figure 2(d)).

Cooling medium flow through cooling element 27 can introduced through a first port, and exit through a second port (Figure 2(e)).

A variety of different cooling mediums can be used including but not limited to gas, cooled air, refrigerated air, compressed air, freon, water, alcohol, and the like. Additionally, cooling element 27 can be incorporated into the walls defining primary antenna 14, and may also be positioned at the exterior of primary antenna 14. The desired cooling affect can be achieved without recirculation of the cooling medium. A chiller can also be utilized. The combination of flow rate of cooling medium and temperature is important to achieve a desired level of cooling.

As the amount of cooling increases, the more RF energy effects can be distributed over a larger area. Cooling is provided and controlled until the end of the ablation, at which time the tissue adjacent to antennas 14 and 16 is then ablated. The RF radiation effect on tissue is controlled by the cooling conductive effect.

Cooling element 27 can also be included with secondary antennas 16, as implemented with primary antenna 14.

Electromagnetic energy delivered through primary or secondary antennas 14 or 16 causes the tissue adjacent to the antenna with the ablation energy delivery surface to heat, and return the heat to the antenna 14 and 16. As more heat is applied and returned, the charring effect of antenna 14 and 16 increases. This can result in a loss of electromagnetic energy conductivity through the antennas. The inclusion of cooling element 27 does not affect the effective delivery of electromagnetic energy to a targeted mass. Cooling element 27 permits the entire targeted mass to be ablated while reducing or eliminating the heating of adjacent tissue to antennas 14 and 16. Cooling is only necessary where there is an exposed antenna 14 and 16 surface.

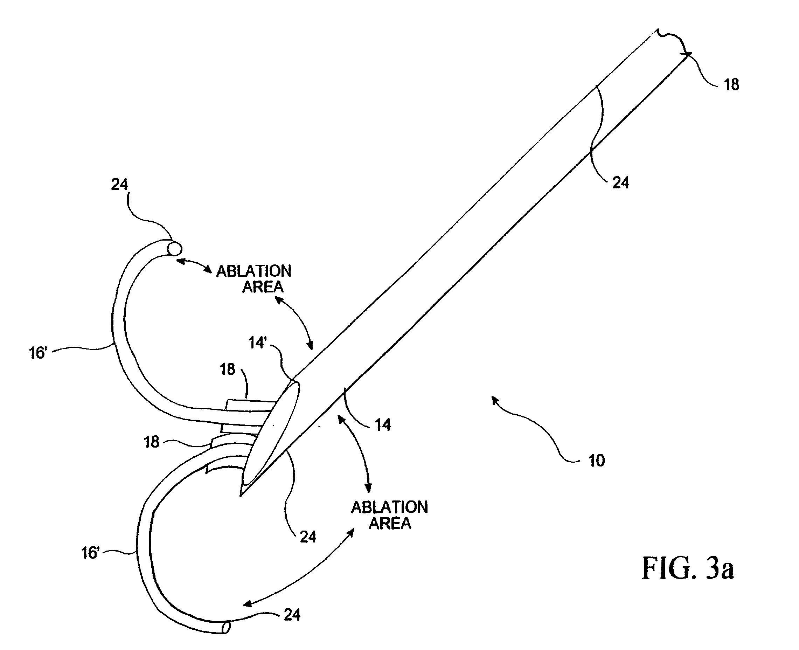

As illustrated in Figure 3(a), ablation treatment device can include two or more secondary antennas 16 which can be independently or dependently laterally deployed along different positions along the longitudinal axis of primary antenna 14. Each secondary antenna 16 is advanced out of a separate aperture 26 formed in the body of primary antenna 14. Multiple secondary antennas 16 can all be introduced along the same planes, a plurality of planes or a combination of both.

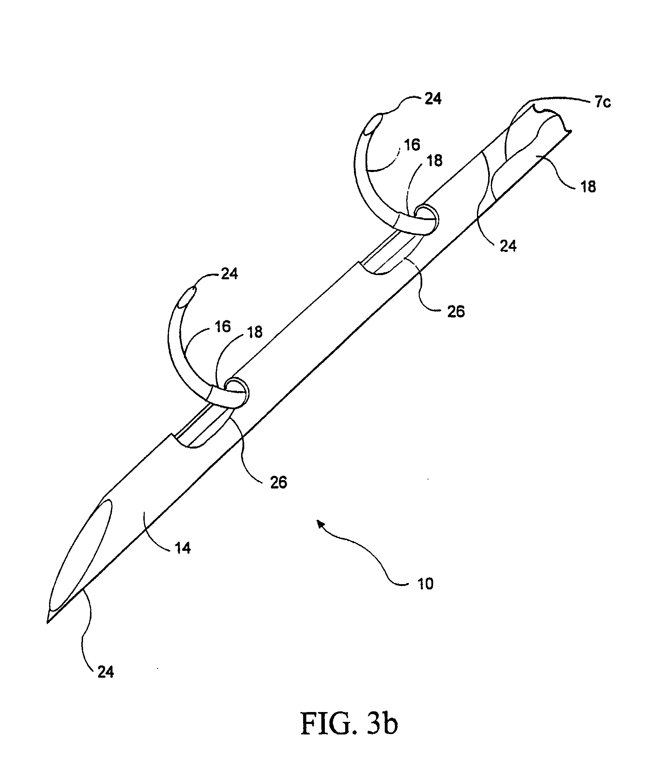

In Figure 3(b), two secondary antennas 16 are each deployed out of distal end 14' and introduced into selected tissue mass 28. Secondary antennas 16 form a plane and the area of ablation extends between the ablation surfaces of primary and secondary antennas 14 and 16. Primary antenna 14 can be introduced in an adjacent relation∼o∼ to selected tissue mass 28. This particular deployment is particularly useful for small selected tissue masses 28, or where piercing selected tissue mass 28 is not desirable. Primary antenna 14 can be rotated, with secondary antennas I 6 retracted into a central lumen of primary antenna 14, and another ablation volume defined between the two secondary antennas I 6 is created. Further, primary electrode 14 can be withdrawn from its initial position adjacent to selected tissue mass 28, repositioned to another position adjacent to selected tissue mass 28, and secondary antennas 16 deployed to begin another ablation cycle. Any variety of different positionings may be utilized to create a desired ablation geometry for selected tissue mass of different geometries and sizes.

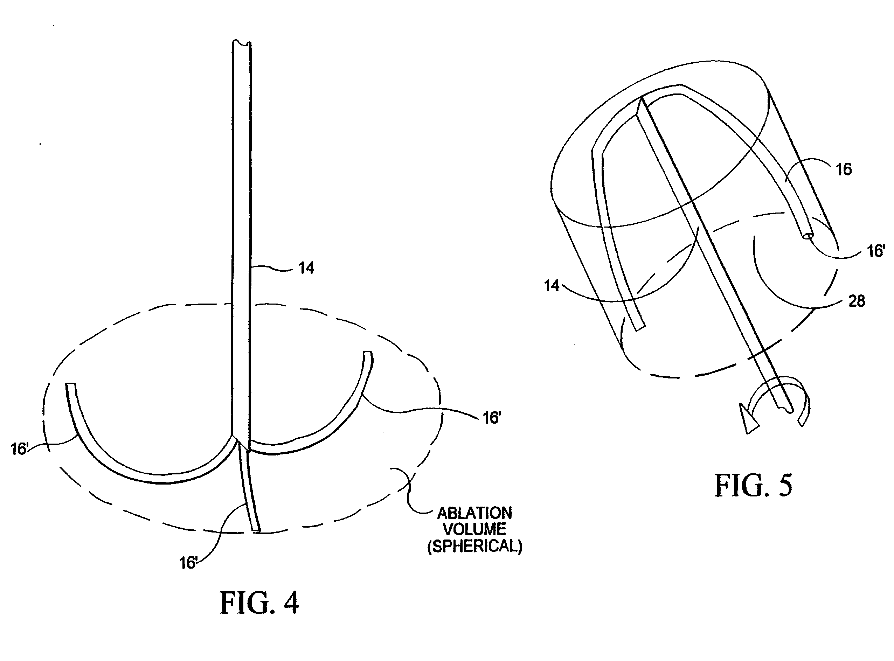

The creation of a spherical ablation is illustrated in Figure 4, and the creation of a cylindrical ablation is illustrated in Figure 5.

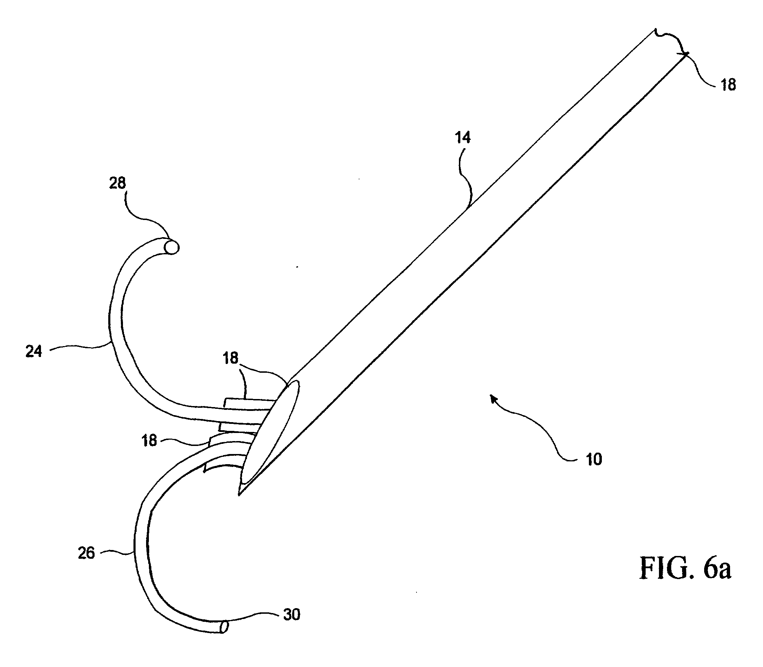

In Figure 6(a), probes 24 and 26 are each deployed out of the distal end of electrode 12 and introduced into the selected tissue mass. Probes 24 and 26 form a plane.

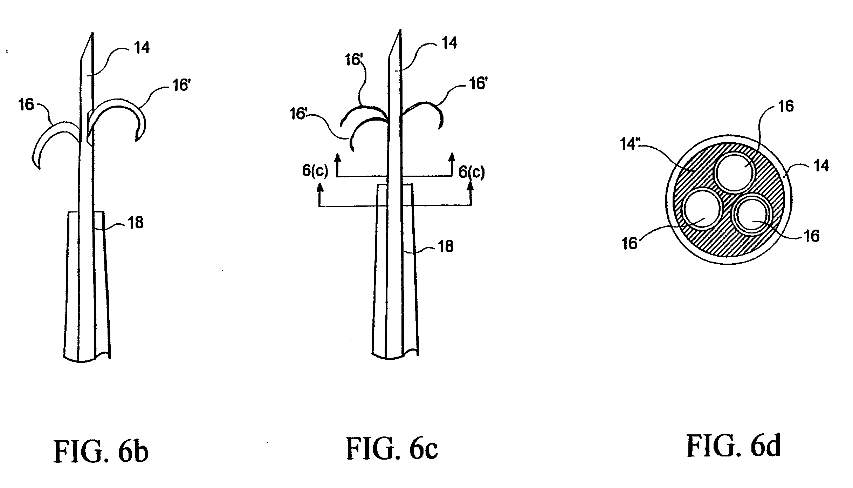

Antennas 16 can serve the additional function of anchoring multiple antenna device 12 in a selected mass, as illustrated in Figures 6(b) and 6(c). In Figure 6(b) one or both antennas 16 are used to anchor and primary antenna or position trocar 14. Further, one or both antennas 16 are also used to ablate tissue. In Figure 6(c), three antennas are deployed and anchor primary antenna or trocar 14.

Figure 6(d) illustrates the infusion capability of multiple antenna device 12. Three antennas 16 are positioned in a central lumen 14" of trocar 14. One or more of the antennas 16 can also include a central lumen coupled to an infusion source. Central lumen 14" is coupled to an infusion source and delivers a variety of infusion mediums to selected places both within and outside of the targeted ablation mass. Suitable infusion mediums include but are not limited to, therapeutic agents, conductivity enhancement mediums, contrast agents or dyes, and the like. An example of a therapeutic agent is a chemotherapeutic agent.

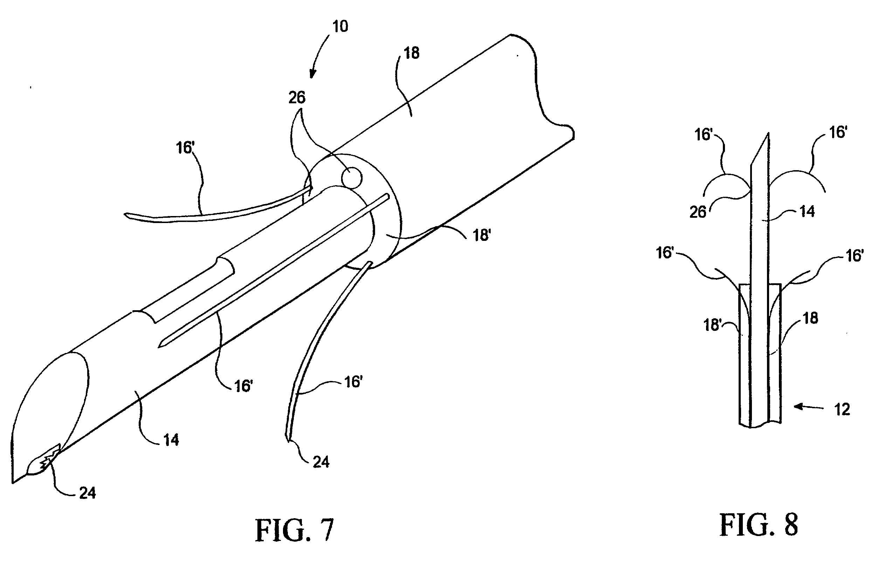

As shown in Figure 7 insulation sleeve 18 can include one or more lumens for receiving secondary probes 24, 26 as well as additional probes which are deployed out of a distal end of insulation sleeve 18.

Figure 8 illustrates four probes introduced out of different sidewall ports formed in the body of electrode 12. Some or all of the probes provide an anchoring function.

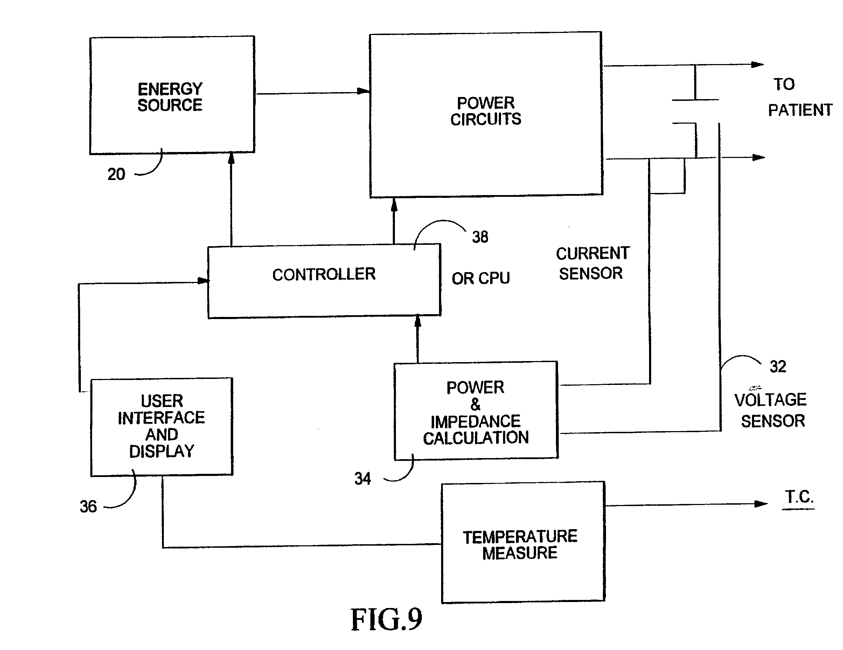

Referring now to Figure 9 current delivered through primary and secondary antennas 14 and 16 is measured by current sensor 30. Voltage is measured by voltage sensor 32. Impedance and power are then calculated at power and impedance calculation device 34. These values can then be displayed at user interface and display 36. Signals representative of power and impedance values are received by controller 38.

In one embodiment, an ablation apparatus includes a handpiece, an electrode extending from a handpiece distal end, a probe, a thermal sensor and an energy source. The electrode includes a distal end and a lumen, a cooling medium inlet conduit and a cooling medium exit conduit. Both conduits extend through the electrode lumen to an electrode distal end. A sidewall port, isolated from a cooling medium flowing in the inlet and outlet conduits, is formed in the electrode. The probe is at least partially positioned in the electrode lumen and configured to be advanced and retracted in and out of the sidewall aperture. The thermal sensor is supported by the probe. The electrode is coupled to an energy source.

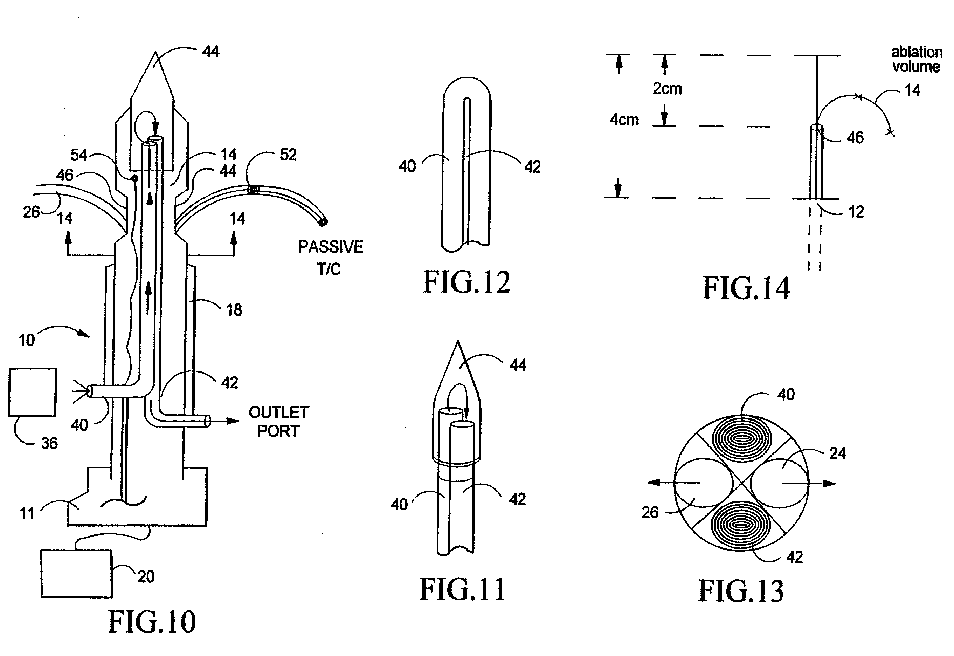

As shown in Figure 10, an ablation apparatus 10 includes a handpiece 11, an electrode 14, a cooling medium inlet conduit 40, a cooling medium outlet conduit 42 and a cap 44, with tapered distal end, that create a closed loop cooling system. A variety of different cooling mediums can be used including but not limited to gas, cooled air, refrigerated air, compressed air, freon, water, alcohol, saline and the like. A first sidewall port 46 is formed in a sidewall of antenna 14. A second sidewall port 48 may also be included. First and second sidewall ports can be windows formed in antenna 14 which create a mechanical weak spot in antenna 14. A first probe 24 is positioned in an electrode lumen and capable of being advanced and retracted in and out of first sidewall port 20. An optional second probe 26 is also positioned in the electrode lumen and is capable of being advanced and retracted to a selected tissue ablation side through second sidewall port 48.

Antenna 14 has an exterior ablation energy delivery surface which delivers electromagnetic energy to the selected tissue ablation mass, and may have a tapered or sharpened distal end. For the ablation of tumors, antenna 14 can have an exterior ablation energy delivery surface length of 0.25 inches or less, and an outer diameter for antenna 14 of about 0.072 inches or less.

Each probe 24 and 26 can be formed of a variety of materials, including but not limited to stainless steel, shaped memory metals and the like. The size of probes 24 and 26 vary depending on the medical application. For the treatment of tumors, probes 24 and 26 have a length extending from the sidewall ports into tissue of 3 cm or less. A first sensor 50 can be supported by probe 24 on an interior or exterior surface. First sensor 50 is preferably positioned at a distal end of probe 24. A second sensor 52 may be positioned on probe 24 somewhere intermediate between an exterior surface of antenna 14 and the distal end of probe 24. Preferably, second sensor 50 is located at a position where it can sense temperature at a midpoint in a selected tissue ablation volume. Second sensor 52 is useful to determine if probe 24 has encountered an obstruction, such as a blood vessel, to the ablation process. If first sensor 50 measures a higher temperature than second sensor 52, then this can indicate that second sensor 52 is close to a circulatory vessel. When this occurs, ablation energy is carried away by the vessel. Similarly, second probe 26 can also include one or more sensors. A third sensor 54 can be positioned at an exterior surface of antenna 14.

Sensors 50, 52 and 54 permit accurate measurement of temperature at a tissue site in order to determine, (i) the extent of ablation, (ii) the amount of ablation, (iii) whether or not further ablation is needed and (iv) the boundary or periphery of the ablated mass. Further, sensors 50, 52 and 54 prevent non-targeted tissue from being destroyed or ablated.

Sensors 50, 52 and 54 are of conventional design, including but not limited to thermistors, thermocouples, resistive wires, and the like. Suitable thermal sensors 24 include a T type thermocouple with copper constantene, J type, E type, K type, fiber optics, resistive wires, thermocouple IR detectors, and the like. Sensors 50, 52 and 54 need not be thermal sensors.

Sensors 50, 52 and 54 measure temperature and/or impedance to permit monitoring and a desired level of ablation to be achieved without destroying too much tissue. This reduces damage to tissue surrounding the targeted mass to be ablated. By monitoring the temperature at various points within the interior of the selected tissue mass, a determination of the selected tissue mass periphery can be made, as well as a determination of when ablation is complete. If at any time sensor 50, 52 or 54 determines that a desired ablation temperature is exceeded, then an appropriate feedback signal is received at energy source 20 which then regulates the amount of energy delivered to antenna 14, as more fully explained hereafter.

Antenna 14 is coupled to an electromagnetic energy source 20 by wiring, soldering, connection to a common couplet, and the like. Antenna 14 can be independently coupled to electromagnetic energy source 2- from probes 24 and 26. Antenna 14, and probes 24 and 26 may be multiplexed so that when energy is delivered to antenna 14 it is not delivered to probes 24 and 26. Electromagnetic energy power source can be an RF source, microwave source, shortwave source, and the like.

Antenna 14 is constructed to be rigid enough so that it can be introduced percutaneously or laparoscopically through tissue without an introducer. The actual length of antenna 14 depends on the location of the selected tissue mass to be ablated, its distance from the skin, its accessibility as well as whether or not the physician chooses a laparoscopic, percutaneous or other procedure. Suitable lengths include but are not limited to 17.5 cm, 25.0 cm. and 30.0 cm. Antenna 14, can be introduced through a guide to the selected tissue ablation site.

An insulation sleeve 18 can be positioned in a surrounding relationship to an exterior surface of antenna 14. Insulation sleeve 18 can be moveable along electrode's 12 exterior surface in order to provide a variable length ablation energy delivery surface.

In one embodiment, insulation sleeve 18 can comprise a polyimide material. A sensor may be positioned on top of polyimide insulation sleeve 18. Polyamide insulation sleeve 18 is semi-rigid. The sensor can lay down substantially along the entire length of polyimide insulation sleeve 18. Handpiece 11 can serve the function of a handpiece and include markings to show the length of insulation sleeve 18 and the length of electrode's 12 exposed ablation energy delivery surface.

Referring now to Figure 11, cap 44 is illustrated as creating a closed loop cooling medium flow channel. Cap 44 is secured to the distal ends of conduits 40 and 42 by a variety of means, including but not limited to welding, soldering, application of an epoxy, and the like. Cap 44 can have a step which is secured to the distal end of antenna 14 by soldering, welding, press sit and the like. Instead of cap 44, a "U' joint can be formed at the distal ends of conduits 40 and 42, as shown in Figure12.

Referring to Figure 13, only a portion of electrode has an interface with cooling medium inlet conduit 40. However, the diameters of cooling medium inlet conduit 40 and antenna 14 are dimensioned so that a tissue interface formed adjacent to the exterior surface of antenna 14 does not become sufficiently desiccated and charred to prevent the transfer of energy through the selected tissue ablation site to the periphery of the site.

The creation of a 4 cm diameter spherical ablation is illustrated in Figure 14. A 4 cm ablation energy delivery surface of antenna 14 is exposed.

Electromagnetic energy delivered by antenna 14 causes the electrode/tissue interface at the electrode ablation delivery surface to heat, and return the heat to antenna 14. As more heat is applied and returned, the charring effect antenna 14 increases. This can result in a loss of electromagnetic energy conductivity through the selected tissue site. The inclusion of cooling with antenna 14 does not affect the effective delivery of electromagnetic energy to the selected tissue ablation site. Cooling permits the entire selected tissue ablation site to be ablated while reducing or eliminating the heating of the electrode/tissue interface tissue.

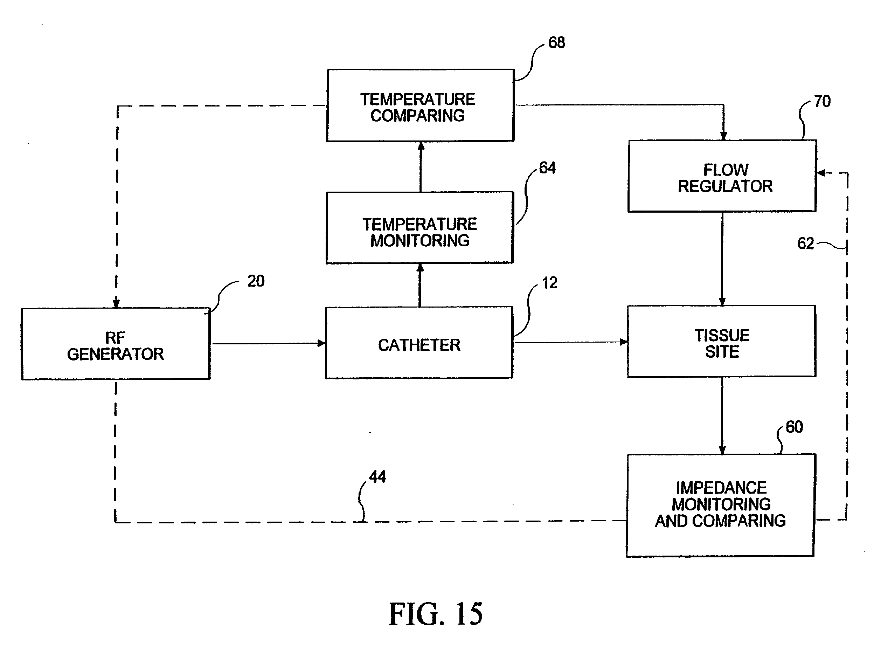

Figure 15 illustrates a block diagram of a temperature/impedance feedback system that can be used to control cooling medium flow rate through antenna 14. Electromagnetic energy is delivered to antenna 14 by energy source 34, and applied to tissue. A monitor 60 ascertains tissue impedance, based on the energy delivered to tissue, and compares the measured impedance value to a set value. if the measured impedance exceeds the set value a disabling signal 62 is transmitted to energy source 20, ceasing further delivery of energy to antenna 14. If measured impedance is within acceptable limits, energy continues to be applied to the tissue. During the application of energy to tissue sensor 64 measures the temperature of tissue and/or antenna 14. A comparator 68 receives a signal representative of the measured temperature and compares this value to a pre-set signal representative of the desired temperature. Comparator 68 sends a signal to a flow regulator 70 representing a need for a higher cooling medium flow rate, if the tissue temperature is too high, or to maintain the flow rate if the temperature has not exceeded the desired temperature.

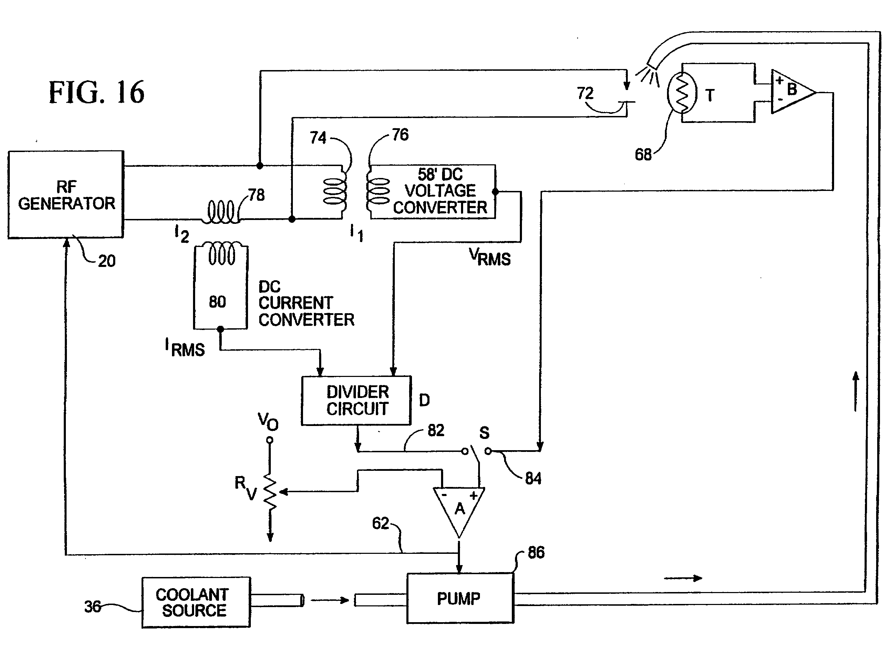

Referring now to Figure 16, energy source 20 is coupled to antenna 14, to apply a biologically safe voltage to the selected tissue site. Both electrodes 14 and 72 are connected to a primary side of transformer windings 74 and 76. The common primary winding 74, 76 is magnetically coupled with a transformer core to secondary windings 78 and 80.

The primary windings 74 of the first transformer t₁ couple the output voltage of ablation apparatus 10 to the secondary windings 78. The primary windings 60 of the second transformer t₂ couple the output current of ablation apparatus 10 to the secondary windings 80.

Measuring circuits determine the root mean square (RMS) values or magnitudes of the current and voltage. These values, represented as voltages, are inputted to a diving circuit D to geometrically calculate, by dividing the RMS voltage value by the RMS current value, the impedance of the tissue site at sensor 68.

The output voltage of the divider circuit D is presented at the positive (+) input terminal of comparator A. A voltage source Vₒ supplies a voltage across the variable resistor Rᵥ, thus allowing one to manually adjust the voltage presented at the negative input of comparator A. This voltage represents a maximum impedance value beyond which power will not be applied to antenna 14. Specifically, once the tissue is heated to a temperature corresponding to an impedance value greater than the maximum cut-off impedance, energy source 20 stops supplying energy to antenna 14. Comparator A can be of any of a commercially available type that is able to control the amplitude or pulse width modulation of energy source 20.

The flow rate of cooling medium can be controlled based on the tissue impedance, as represented by signal 82, or based on tissue temperature, as represented by signal 84. In one embodiment, the switch S is activated to allow the impedance signal 82 to enter the positive (+) input terminal of comparator A. This signal along with the reference voltage applied to the negative (-) input terminal actuates comparator A to produce an output signal. If the selected tissue ablation site is heated to a biologically damaging temperature, the tissue impedance will exceed a selected impedance value seen at the negative (-) input terminal, thereby generating disabling signal 62 to disable energy source 20, ceasing the power supplied to antenna 14.

The output signal of comparator A can be communicated to a pump 86. If the temperature of the selected tissue ablation site is too high, despite the tissue impedance falling within acceptable limits, pump 86 adjusts the rate of cooling medium flow applied to antenna 14 to decrease the temperature of antenna 14. The output signal of comparator A may either disable energy source's 20 energy output, depending on the tissue temperature as reflected by its impedance, or cool antenna 14 or perform both operations simultaneously.

The foregoing description of a preferred embodiment of the invention has been presented for purposes of illustration and description. It is not intended to be exhaustive or to limit the invention to the precise forms disclosed. Obviously, many modifications and variations will be apparent to practitioners skilled in this art. It is intended that the scope of the invention be defined by the following claims and their equivalents.

## Claims

1. An ablation treatment apparatus, comprising:
a multiple antenna device including a primary antenna with a lumen, a longitudinal axis and an ablative surface area of length L₁, and a secondary antenna positioned in the primary antenna as the primary antenna is introduced through tissue and a secondary antenna distal end is deployed at a selected tissue site from the primary antenna lumen in a lateral direction relative to the longitudinal axis;
a sensor at least partially positioned at an exterior of the secondary antenna distal end at a distance L2 from the primary antenna along the secondary antenna distal end, wherein L, is at least equal to 1/3 L₁; and an energy source coupled to the primary antenna.

2. The apparatus of claim 1, wherein L₂ is at least equal to 1/2 L₁.

3. The apparatus of claim 1, wherein L₂ is at least equal to L₁.

4. The apparatus of claim 1, further comprising:
a sensor monitoring device coupled to the sensor.

5. The apparatus of claim 4, wherein the sensor monitoring device is a temperature monitoring device.

6. The apparatus of claim 1, further comprising:
a feedback control device coupled to the energy source and the sensor, wherein the feedback control device is responsive to a detected characteristic from the sensor and provides a delivery of electromagnetic energy output from the energy source the primary antenna.

7. The apparatus or claim 1, wherein the secondary antenna is an inactive electrode and the primary antenna is an active electrode.

8. The apparatus of claim 1, wherein the secondary antenna distal end is structurally less rigid than the primary antenna.

9. The apparatus of claim 1, wherein the apparatus is switchable between bipolar and monopolar operation.

10. The apparatus of claim 1, wherein the primary antenna is hollow and coupled to an infusion medium source to receive an infusion medium.

11. The apparatus of claim 1, wherein the primary antenna has an ablation surface with a length that is at least 20% of a length of an ablation surface of the secondary antenna.

12. The apparatus of claim 1, the primary antenna has an ablation surface with a length that is at least one-third of a length of an ablation surface of the secondary antenna.

13. The apparatus of claim 1, wherein the primary antenna has an ablation surface with a length that is at least one-half of a length of an ablation surface of the secondary antenna.

14. The apparatus of claim 1, wherein two secondary electrodes are provided and laterally deployed from the primary antenna each of the primary and secondary antennas having an ablation surface to create an ablation volume between the ablation surfaces.

15. The apparatus of claim 1 wherein three secondary electrodes are provided and laterally deployed from the primary antenna, each of the primary and secondary antennas having an ablation surface to create an ablation volume between the ablation surfaces.

16. The apparatus of claim 1, further comprising:
an insulation sleeve positioned in a surrounding relationship around at least a portion of an exterior of the primary antenna.

17. The apparatus of claim 16, wherein the insulation sleeve is adjustably moveable along an exterior of the primary antenna.

18. The apparatus of claim 1, further comprising:
an insulation sleeve positioned in a surrounding relationship around at least a portion of an exterior of the secondary antenna.

19. The apparatus of claim 13, the insulation sleeve is adjustably moveable along an exterior of the secondary antenna.

20. The apparatus of claim 1, further including a ground pad electrode with the primary antenna operating in a monopolar mode.

21. The apparatus of claim I, wherein the primary antenna is an RF antenna.

22. The apparatus of claim 1. wherein the primary antenna is a microwave antenna.

23. Tile apparatus of claim 1, wherein the secondary antenna is an inactive antenna.

24. The apparatus of claim 1, wherein the primary antenna is hollow and coupled to an infusion medium source to receive an infusion medium.

25. The apparatus of claim 1, wherein the secondary antenna distal end is less structurally rigid than the primary antenna.

26. A method for creating an ablation volume in a selected tissue mass, comprising:
providing an ablation device with a primary antenna with an electromagnetic energy delivery surface, a secondary antenna with a distal end, a sensor at least partially positioned at an exterior of the secondary antenna distal end, and an energy source coupled to the primary antenna;
inserting the primary antenna into the selected tissue mass with the secondary antenna distal end positioned in the primary antenna lumen;
advancing the secondary antenna distal end out of the primary antenna lumen and into the selected tissue mass in a lateral direction relative to a longitudinal axis of the primary antenna;
delivering electromagnetic energy from the primary antenna ablation surface to the selected tissue mass;
measuring a temperature of the selected tissue mass at the sensor; and
creating an ablation volume in the selected tissue mass.

27. The method of claim 26, wherein the secondary antenna is a inactive antenna.

28. The method of claim 26, wherein the secondary antenna distal end is an electromagnetic energy delivery surface.

29. The method of claim 26, wherein the secondary antenna distal end is advanced out of a distal end of the primary antenna.

30. The method of claim 26, wherein the ablation device is operated in a monopolar mode.

31. The method of claim 26, wherein the ablation device is operated in a bipolar mode.

32. The method of claim 26, wherein the ablation device is switchable between monopolar and bipolar operation.

33. The method of claim 26, wherein the sensor measures temperature.

34. An ablation treatment apparatus, comprising:
an electromagnetic energy source;
a trocar including a distal end, and a hollow lumen extending along a longitudinal axis of the trocar;
a multiple antenna ablation device including three or more antennas positioned in the trocar lumen as the trocar is introduced through tissue and the antennas are deployable from the trocar lumen in a lateral direction relative to the longitudinal axis at a selected tissue mass, each of a deployed antenna having an electromagnetic energy delivery surface size sufficient to create a volumetric ablation between the deployed antennas without impeding out a deployed antenna when 10 to 50 watts of electromagnetic energy is delivered from the electromagnetic energy source to the multiple antenna ablation device; and
at least one cable coupling the multiple antenna ablation device to the electromagnetic energy source.

35. The apparatus of claim 34, wherein each of the deployed antenna has an electromagnetic energy delivery surface size sufficient to create a volumetric ablation between the deployed antennas without impeding out a deployed antenna when 10 to 30 watts of electromagnetic energy is delivered from the electromagnetic energy source to the multiple antenna ablation device.

36. The apparatus of claim 34, wherein each of the deployed antenna has an electromagnetic energy delivery surface size sufficient to create a volumetric ablation between the deployed antennas without impeding out a deployed antenna when 15 to 20 watts of electromagnetic energy is delivered from the electromagnetic energy source to the multiple antenna ablation device.

37. The apparatus of claim 34, wherein each of the antennas is coupled to the electromagnetic energy source.

38. The apparatus of claim 34, wherein the trocar has an outer diameter no greater than 13 gauge.

39. The apparatus of claim 34, wherein the trocar has an outer diameter no greater than 14 gauge.

40. The apparatus of claim 34, wherein the trocar has an outer diameter no greater than 15 gauge.

41. The apparatus of claim 34, wherein four antennas are deployed from the trocar at the selected tissue mass.

42. The apparatus of claim 34, wherein five antennas are deployed from the trocar at the selected tissue mass.

43. The apparatus of claim 34, wherein six antennas are deployed from the trocar at the selected tissue mass.

44. The apparatus of claim 34, wherein at least two of the antennas are deployed out of the trocar distal end.

45. The apparatus of claim 34, wherein the trocar includes one or more side ports formed in a body of the trocar.

46. The apparatus of claim 34, wherein at least one antenna is deployed into the selected tissue mass from the trocar distal end and at least one antenna is deployed into the selected tissue mass from a side port.

47. The apparatus of claim 34, wherein the antennas are RF electrodes and the electromagnetic energy source is an RF energy source.

48. The apparatus of claim 34, wherein the multiple antenna ablation device operates in a monopolar mode.

49. The apparatus of claim 34, wherein the multiple antenna ablation device operates in a bipolar mode.

50. The apparatus of claim 34, wherein the apparatus is switchable between bipolar and monopolar operation.

51. The apparatus of claim 34, wherein at least a portion of a distal end of each antenna is constructed to be structurally less rigid than the trocar.

52. The apparatus of claim 34, further comprising:
a sensor at least partially positioned on an exterior surface of an antenna.

53. The apparatus of claim 34, further comprising:
an insulation layer positioned in a surrounding relationship around at least a portion of an exterior of the trocar.

54. The apparatus of claim 53, wherein a distal end of the insulation layer is removed from the distal end of the trocar and create an electromagnetic energy delivery surface at the distal end of the trocar.

55. The apparatus of claim 34, wherein the trocar lumen is coupled to an infusion medium source to receive an infusion medium.

56. An ablation treatment apparatus, comprising:
an electromagnetic energy source;
a trocar including a distal end, and a hollow lumen extending along a longitudinal axis of the trocar;
a multiple antenna ablation device including a plurality of antennas positioned in the trocar lumen as the trocar is introduced through tissue and the plurality of antennas is deployable from the trocar lumen in a lateral direction relative to the longitudinal axis at a selected tissue mass, wherein the plurality of antennas includes a sufficient number of antennas to create an ablation volume between the antennas in the selected tissue site without impeding out the plurality of antennas when 10 to 50 watts of electromagnetic energy is delivered from the electromagnetic energy source to the plurality of antennas; and
at least one cable coupling the multiple antenna ablation device to the electromagnetic energy source.

57. The apparatus of claim 56, wherein the trocar has an outer diameter no greater than 15 gauge.

58. The apparatus of claim 56, wherein the multiple antenna ablation device is an RF device.

59. The apparatus of claim 58, wherein the multiple antenna ablation device operates in a monopolar mode.

60. The apparatus of claim 58, wherein the multiple antenna ablation device operates in a bipolar mode.

61. The apparatus of claim 58, wherein the multiple antenna ablation device is switchable between bipolar and monopolar operation.

62. A method for creating a volumetric ablation in a selected tissue mass, comprising:
providing a multiple antenna ablation apparatus including a trocar with a trocar lumen, a plurality of antennas deployable from the lumen, and an electromagnetic energy source coupled to the plurality of antennas;
inserting the trocar into the selected tissue mass with the plurality of antennas positioned in the trocar lumen;
advancing the plurality of antennas from the trocar lumen in a lateral direction relative to a longitudinal axis of the trocar into the selected tissue mass;
delivering 10 to 50 watts of electromagnetic energy from the electromagnetic energy source to the plurality of antennas without impeding out an antenna of the plurality of antennas; and
creating the volumetric ablation in the selected tissue mass.

63. The method of claim 62, wherein the ablation volume is a spheroid geometry.

64. The method of claim 62, wherein the ablation volume is a spherical geometry.

65. The method of claim 62, wherein the ablation volume is a partial spherical geometry.

66. The method of claim 62, wherein the ablation volume is formed continuously between adjacently deployed antennas of the plurality.

67. The method of claim 62, wherein the trocar has an outer diameter no greater than 15 gauge.

68. The method of claim 62, wherein each of an antenna of the plurality has an electromagnetic energy delivery surface size sufficient to receive 10 to 50 watts of electromagnetic energy from the electromagnetic energy source.
